# EUROPEAN PATENT SPECIFICATION

(11) **EP 1 809 274 B1**
(45) Date of publication and mention of the grant of the patent: **28.04.2010**
(21) Application number: 05777365.7
(22) Date of filing: 07.09.2005
(51) Int. Cl.: A61K 31/282, A61K 9/02, A61K 47/42, A61K 47/26

(54) **PHARMACEUTICAL COMPOSITION FOR RECTAL OR VAGINAL APPLICATION COMPRISING A PLATINUM COMPLEX**
PHARMAZEUTISCHE ZUSAMMENSETZUNG ZUR REKTALEN ODER VAGINALEN ANWENDUNG MIT EINEM PLATINKOMPLEX
COMPOSITION PHARMACEUTIQUE DESTINEE A UNE APPLICATION RECTALE OU VAGINALE, CONTENANT UN COMPLEXE À BASE DE PLATINE

(30) Priority: 08.09.2004 CZ 20040945
(43) Date of publication of application: 25.07.2007
(73) Proprietor: Pliva-Lachema A.S., 621 33 Brno (CZ)
(72) Inventor: FRANC, Ales, 612 00 Brno (CZ); SOVA, Petr, 500 02 Hradec Králové (CZ)
(74) Representative: Pavlica, Tomas
(86) International application number: PCT/CZ2005/000068
(87) International publication number: WO 2006/026935

(56) References cited:
- EP-A- 1 523 984
- US-A- 6 136 336
- CHEN Q M ET AL: "The stability of carboplatin suppository" CHINESE PHARMACEUTICAL JOURNAL 2000 CHINA, vol. 35, no. 3, 2000, pages 168-171, XP009069276 ISSN: 1001-2494
- MATSUHISA T ET AL: "Preparation of cisplatin suppository and its clinical evaluation" IRYO - JAPANESE JOURNAL OF NATIONAL MEDICAL SERVICES 1994 JAPAN, vol. 48, no. 8, 1994, pages 612-616, XP009069277 ISSN: 0021-1699
- WAKATSUKI KAZUO ET AL: "Effects of irradiation combined with cis-diamminedichloroplatinum (CDDP) suppository in rabbit VX2 rectal tumors." WORLD JOURNAL OF SURGERY. MAR 2005, vol. 29, no. 3, March 2005 (2005-03), pages 388-395, XP002389724 ISSN: 0364-2313

## Description

### Field of Invention

This invention relates to a pharmaceutical composition for rectal or vaginal application, method of manufacturing and use as medicament thereof

### Background of the Invention

Platinum cytostatics, such as e.g. cisplatinum, carboplatinum or oxaliplatinum, are being used for many years in the treatment of solid tumors. However, these platinum cytostatics cannot be effectively applied orally because they are only sparingly soluble in waster, unstable in the acidic medium of the stomach, and they are absorbed by the organism only with difficulty. Since in comparison with the alternative parenteral administration the oral application is much bearable and comfortable for the patient, there has been a need in the art for cytostatically effective platinum complexes whose solubility in water and absorbability by the organism would enable their oral application. Such orally applicable complexes of tetravalent platinum have been described in EP 0 328 274 and EP 0 423 707. Stability of these substances in an acidic medium, together with their physicochemical properties are favorable factors for their absorption by mucosa of the gastrointestinal tract. Nevertheless, in the form of particles, these complexes of tetravalent platinum have a high electrostatic charge that hampers their continuous processing to give a solid dosage form such as e.g. tablets. This disadvantage has been overcome by obtaining the mentioned platinum complexes in the form of inclusion complexes with cyclodextrins, their conversion into organic solution and subsequent lyophilization as described in WO 99/61451, or by processing using the wet granulation method as described in patent applications CZ 2003-915 and CZ 2004-235. According to the above patent applications, it is possible to use up to 350 mg of the active substance in a dose unit and to achieve enterosolvent and controlled release of the active pharmaceutical composition in the form of an oral solid dosage form.

Another possible application of platinum cytostatics is the rectal application, in which the resorbed drug avoids the decomposing aggressive medium of the gastrointestinal tract and after absorption by the rectal mucosa it passes via the portal system directly into the systemic blood circulation. The possibility of rectal application of a tetravalent platinum complex has been described in US 6 033 683. Another possibility of application of platinum cytostatics is based on their administration in the form of vaginal globules in diseases of urogenital tract in women. The platinum cytostatic is released in the area of vagina and urethra and this local application enables the active substance to act directly at the tumor site.

The document US 6,136,336 teaches a method of increasing the aqueous solubility and bioavailabity of the platinum antitumor complex bis-acetato-ammine-dichloro-cyclohexylamin platinum (IV).

The DAB9-Kommentar 1986 classifies the base comprising gelatin, water and glycerol as commonly used for the preparation of the vaginal ovule.

Recently, there is an endeavor to apply oral complexes of tetravalent platinum of the type described in EP 0 328 274 and EP 0 423 707 also per rectum or per vagina in order to achieve a more potent effect of the mentioned complexes directly at the site of the tumor. Thus, the objective of the invention is to prepare a stable pharmaceutical composition, containing the mentioned tetravalent platinum complexes, that could be suitable for rectal and vaginal application.

This objective has been achieved by the pharmaceutical composition and the method of preparation thereof according to this invention.

### Summary of the Invention

The invention relates to a pharmaceutical composition for rectal or vaginal application, **characterized in that** it contains as active substance a platinum complex of formula II 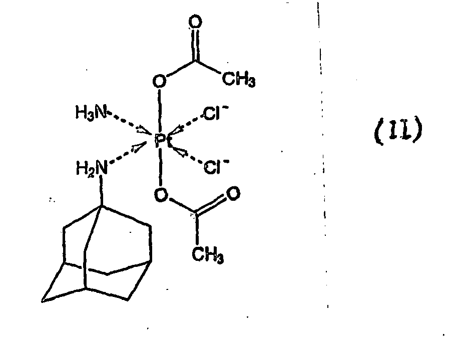 and a hydrophilic gel-forming base, comprising gelatin, water and glycerol.

The pharmaceutical composition according to this invention advantageously contains at least one monosaccharide and/or disaccharide and/or polysaccharide.

The active substance in the pharmaceutical composition advantageously has such particle size distribution that 100 % of the particles of the active substance are smaller than or equal to 100 micrometers, advantageously 80 % of the particles of the active substance are smaller than or equal to 50 micrometers.

The pharmaceutical composition according to the invention advantageously is in the form of a unit dose containing 5 to 500 mg of the active substance, preferably 20 to 200 mg of the active substance.

The pharmaceutical composition according to the invention advantageously is in the form of a rectal suppository or a vaginal globule.

The invention also relates to a method of manufacturing the mentioned pharmaceutical composition, **characterized in that** the active substance is admixed with a thermally liquefied hydrophilic gel-forming base comprising gelatin, water and glycerol, whereupon the obtained mixture is shaped.

In the method according to the invention, the active substance is advantageously admixed with a thermally liquefied hydrophilic gel-forming base, in a mixture with at least one monosaccharide and/or disaccharide and/or polysaccharide with which the active substance had been pre-ground to the desired particle size.

According to the invention, the content of at least one monosaccharide and/or disaccharide and/or polysaccharide in the mixture with the active substance, intended for the pre-grinding, advantageously amounts to at least 20 % by weight, preferably up to 50 % by weight, based on the weight of the active substance.

According to the invention, the active substance is advantageously ground in a mixture with at least one monosaccharide and/or disaccharide and/or polysaccharide to achieve such particle size distribution that 100 % of the particles of the active substance are smaller than or equal to 100 micrometers, 80 % of the particles of the active substance being advantageously smaller than or equal to 50 micrometers.

According to the invention, the admixing of the active substance with the thermally liquefied hydrophilic gel-forming base is advantageously performed in a mixer in which the inner surface, coming into contact with the processed mixture, is a inert material, advantageously made of glass or ceramic, or it is teflon-coated or otherwise non-metallically modified.

According to the invention, the mixture obtained by admixing the active substance with the thermally liquefied hydrophilic gel-forming base is advantageously shaped by casting into rectal suppository or vaginal globule moulds, and then the cast mixture is left to cool down or cooled.

According to the invention, the mixture obtained by admixing the active substance with the molten hydrophilic gel-forming base, is advantageously shaped by casting into rectal suppository or vaginal globule moulds and subsequent leaving to cool down, or cooling, to give rectal suppositories or vaginal globules weighing 0.5 to 6 g.

The present invention also relates to the above defined pharmaceutical composition, or a composition prepared by the above-defined method, for use as a medicament for treatment of tumor diseases.

The pharmaceutical composition according to the invention comprises the mentioned platinum complex of formula II evenly suspended in a hydrophilic gel-forming base. Although the platinum complex of formula II generally is incompatible with currently used pharmaceutically acceptable excipients, it has been surprisingly found that it is well compatible with a combination of components forming the hydrophilic gel-forming base according to the invention. Moreover, this base does not attack, and thus does not decompose, the mentioned platinum complex and enables its effective absorption by the rectal and vaginal mucosa. In grinding of the active substance to the desired particle size, it is possible to perform the grinding in the presence of at least one monosaccharide and/or disaccharide and/or polysaccharide, particularly in the presence of lactose, this compound serving as an auxiliary in the presence of which no substantial decomposition of the active substance takes place. This auxiliary also increases the viscosity of the mixture for preparation of rectal suppository or vaginal globule material, preventing thus sedimentation of the active substance during the preparation, solidification or gel formation. When using this excipient, a mixture of the active substance and the excipient may thus be added already into a solution of the hydrophilic gel-forming base of low viscosity. Dissolution of the excipient in the mentioned solution increases viscosity of the solution, thus inhibiting sedimentation of the active substance. When using the active substance alone, it is necessary to inhibit its sedimentation by adding the active substance to the hydrophilic gel-forming base only after cooling the base to a temperature ranging from about 40 °C to about 50 °C when the base exists already in the gel form.

In the following part the invention will be explained in more detail using individual examples of its execution which however are only illustrative and do not limit its scope. Within the framework of these examples, the platinum complex employed is af-bis(acetato)-b-(1-adamantylamine)-c-ammine-de-dichloroplatinum(IV) complex of the structural formula II.

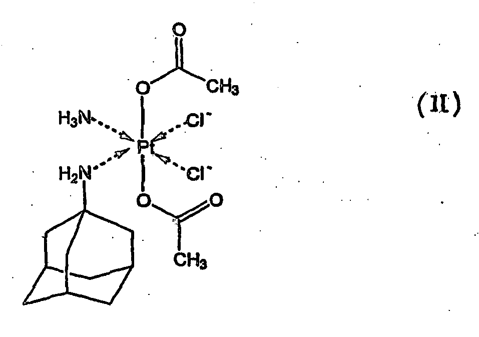

This complex of summary formula C₁₄H₂₆Cl₂N₂O₄Pt and of molecular weight 552.35 contains (acetato)-(1-adamantylamine)-ammine-trichloroplatinum(IV) complex of structural formula Pt(ac)(am)(NH₃)Cl₃ as principal detectable impurity. The specific platinum(IV) complex employed, together with its antitumor effects, is described in the patent document WO 99/61451 where it is denoted as LA 12. It is sparingly soluble in water, its solubility being 0.03 g/100 ml, has a low bulk density amounting to 0.21 g/ml and a low tap density amounting to 0.42 g/ml, and an extremely high electrostatic charge.

### Examples

### Example 1

### Preparation of rectal suppositories

70 parts by weight of glycerol and 10 parts by weight of water are mixed together and the resulting mixture is heated to 70 °C, whereupon 20 parts by weight of gelatin is added and the mixture obtained is slowly stirred for 45 minutes to dissolve the gelatin. Then the mixture is cooled to a temperature ranging from about 40 °C to about 50 °C to obtain a gel into which 20 parts by weight of the platinum(IV) complex LA 12 is added under slow stirring. The stirring is continued until a homogeneous gel suspension is obtained which is then cast into moulds for shaping rectal suppositories, the moulds being pre-hydrophobized by spreading with paraffin oil. The mould content is left to cool to room temperature and the rectal suppositories are taken out and adjusted in blisters.

### Example 2

### Preparation of vaginal globules

70 parts by weight of glycerol and 10 parts by weight of water are mixed together and the resulting mixture is heated to 70 °C, whereupon 20 parts by weight of gelatin is added and the mixture obtained is slowly stirred for 45 minutes to dissolve the gelatin. To this mixture is added, with slow stirring, a mixture of 40 parts by weight of lactose and 20 parts by weight of platinum(IV) complex LA 12, which mixture had been pre-ground in a ball mill in order to satisfy the condition that 80 % of particles of the platinum complex LA 12 are smaller than or equal to 50 micrometers. The stirring at the temperature mentioned is continued until the lactose is dissolved. Then, this mixture is cooled to a temperature ranging from about 40 °C to about 50 °C to obtain a homogeneous gel suspension which is then cast into moulds for shaping vaginal globules, the moulds being pre-hydrophobized by spreading with paraffin oil. The mould content is left to cool to room temperature and the vaginal globules are taken out and adjusted in blisters.

## Claims

1. A pharmaceutical composition for rectal and vaginal application, **characterized in that** it contains as active substance the platinum complex of formula II 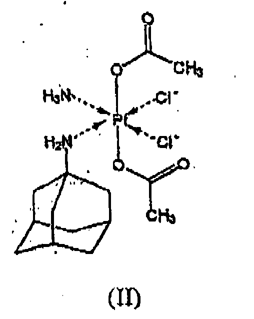 and a hydrophilic gel-forming base, comprising gelatin, water and glycerol.

2. The pharmaceutical composition according to claim 1, **characterized in that** it comprises at least one monosaccharide and/or disaccharide and/or polysaccharide.

3. The pharmaceutical composition according to claim 1 or 2, **characterized in that** the active substance has such particle size distribution that 100 % of particles of the active substance are of size smaller than or equal to 100 micrometers, preferably 80 % of particles of the active substance are of size smaller than or equal to 50 micrometers.

4. A pharmaceutical composition according to any of claims 1 to 3, **characterized in that** it is in the form of a unit dose comprising 5 to 500 mg of the active substance, advantageously 20 to 200 mg of the active substance.

5. A pharmaceutical composition according to any of claims 1 to 4, **characterized in that** it is in the form of a rectal suppository or a vaginal globule.

6. A method of manufacturing pharmaceutical composition according to any of claims 1 to 5, **characterized in that** the active substance is admixed with a thermally liquefied hydrophilic gel-forming base comprising gelatin, water and glycerol, whereupon the mixture obtained is shaped.

7. The method according to claim 6, **characterized in that** a mixture of the active substance with at least one monosaccharide and/or disaccharide and/or polysaccharide, with which the active substance had been pre-ground to obtain the desired particle size, is admixed with thermally liquefied hydrophilic gel-forming base.

8. The method according to claim 7, **characterized in that** the content of at least one monosaccharide and/or disaccharide and/or polysaccharide in the mixture with the active substance intended for the pre-grinding equals to or is higher than 20 % by weight, preferably up to 50 % by weight, based on the weight of the active substance.

9. A method according to claims 7 or 8, **characterized in that** the active substance is ground in a mixture with at lest one monosaccharide and/or disaccharide and/or polysaccharide to obtain such particle size distribution that 100 % of the particles of the active substance are of size smaller than or equal to 100 micrometers, preferably 80 % of the particles of the active substance are of size smaller than or equal to 50 micrometers.

10. A method according to any of claims 6 to 9, **characterized in that** admixing the active substance with the thermally liquefied hydrophilic gel-forming base is performed in a mixer in which the inner surface, coming into contact with the processed mixture, is made of a inert material, advantageously of glass or ceramic, or of a teflon-coated, or otherwise non-metallically modified material.

11. A method according to any of claims 6 to 10, **characterized in that** the mixture, obtained by admixing the active substance with the thermally liquefied hydrophilic gel-forming base, is shaped by casting into moulds for rectal suppositories or vaginal globules, and subsequent leaving to cool down or cooling of the cast mixture.

12. The method according to claim 11, **characterized in that** the mixture, obtained by admixing the active substance with the thermally liquefied hydrophilic gel-forming base, is shaped by casting into moulds for rectal suppositories or vaginal globules, and after leaving to cool down or cooling of the cast mixture, affords rectal suppositories or vaginal globules weighing 0.5 g to 6 g.

13. A pharmaceutical composition according to any of claims 1 to 5 or a pharmaceutical composition prepared by a method according to any of claims 6 to 12, for use as a medicament for treatment of tumor diseases.
Formula for the Abstract (I)

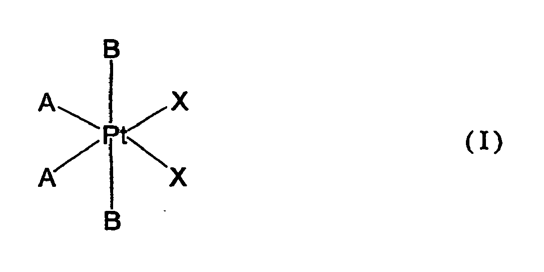

## Patentansprüche

1. Eine pharmazeutische Zusammensetzung zur rektalen und vaginalen Anwendung, **dadurch gekennzeichnet, dass** es als Wirkstoff den Platin-Komplex der Formel II 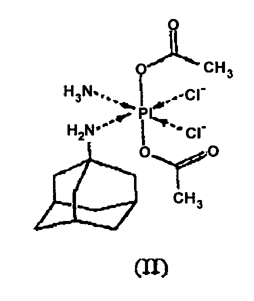 und eine hydrophile, Gel-bildende, aus Gelatine, Wasser und Glycerin bestehende Basis enthält,.

2. Die pharmazeutische Zusammensetzung nach Anspruch 1, **dadurch gekennzeichnet, dass** sie mindestens ein Monosaccharid und/oder Disaccharid und/oder Polysaccharid enthält.

3. Eine pharmazeutische Zusammensetzung nach Anspruch 1 oder 2, **dadurch gekennzeichnet, dass** der Wirkstoff eine solche Teilchengrößenverteilung hat, dass 100 % der Partikel des Wirkstoffs von der Größe kleiner als oder gleich 100 Mikrometer ist, vorzugsweise 80 % der Partikel des Wirkstoffs von der Größe kleiner als oder gleich 50 Mikrometer ist.

4. Eine pharmazeutische Zusammensetzung nach einem der Ansprüche 1 bis 3, **dadurch gekennzeichnet, dass** es in der Form einer Einzeldosis bestehend aus 5 bis 500 mg des Wirkstoffs, vorteilhaft von 20 bis 200 mg des Wirkstoffs ist.

5. Eine pharmazeutische Zusammensetzung nach einem der Ansprüche 1 bis 4, **dadurch gekennzeichnet, dass** es in der Form eines rektalen Suppositor oder eines vaginalen Kügelchens ist.

6. Ein Verfahren zur Herstellung von einer pharmazeutischen Zusammensetzung nach einem der Ansprüche 1 bis 5, **dadurch gekennzeichnet, dass** der Wirkstoff mit einer thermisch verflüssigten, hydrophilen, Gel-bildenden, aus Gelatine, Wasser und Glycerin bestehender Basis gemischt wird, worauf die erhaltene Mischung geformt wird.

7. Die Methode nach Anspruch 6, **dadurch gekennzeichnet, dass** eine Mischung des Wirkstoffs mit mindestens einem Monosaccharid und/oder Disaccharid und/oder Polysaccharid, mit dem der Wirkstoff vorgemahlen wurde, um die gewünschte Partikelgröße zu erhalten, mit thermisch verflüssigten, hydrophilen, Gel-bildenden Basis zugemischt wird.

8. Das Verfahren nach Anspruch 7, **dadurch gekennzeichnet, dass** der Inhalt von mindestens einem Monosaccharid und/oder Disaccharid und/oder Polysaccharid in der Mischung mit dem Wirkstoff für das Vormahlen gleich oder höher als 20 Gew. %, vorzugsweise bis zu 50 Gew. %, bezogen auf das Gewicht des Wirkstoffs, ist.

9. Ein Verfahren nach einem der Ansprüche 7 oder 8, **dadurch gekennzeichnet, dass** der Wirkstoff in einer Mischung mit mindestens einem Monosaccharid und/oder Disaccharid und/oder Polysaccharid gemahlen wird, um solche Korngrößenverteilung zu erhalten, dass 100 % der Partikel des Wirkstoffs von der Größe kleiner als oder gleich 100 Mikrometer, vorzugsweise 80 % der Partikel des Wirkstoffs von der Größe kleiner als oder gleich 50 Mikrometer ist.

10. Ein Verfahren nach einem der Ansprüche 6 bis 9, **dadurch gekennzeichnet, dass** die Zumischung des Wirkstoffs mit der thermisch verflüssigten, hydrophilen, Gel-bildenden Basis in einem Mischer erfolgt, in welchem die innere Oberfläche, die in Kontakt mit der verarbeiteten Mischung kommt, aus einem inerten Material, vorteilhaft aus Glas oder Keramik, oder aus einem Teflon-beschichtetem, oder auf andere Weise nicht metallisch modifiziertem Material erzeugt wurde.

11. Ein Verfahren nach einem der Ansprüche 6 bis 10, **dadurch gekennzeichnet, dass** die Mischung, die durch Zumischung des Wirkstoffs mit der thermisch verflüssigten, hydrophilen Gel-bildenden Basis erhalten wurde, durch Gießen in Formen für die rektalen Suppositorien oder vaginale Kügelchen, und das anschließende Lassen, um abzukühlen, oder Kühlen der gegossenen Mischung geformt wird.

12. Das Verfahren nach Anspruch 11, **dadurch gekennzeichnet, dass** die durch Zumischung der Wirkstoff mit der thermisch verflüssigten, hydrophilen, Gel-bildenden Basis erhaltene Mischung, durch Gießen in Formen für die rektale Suppositorien oder vaginale Kügelchen geformt wird, und nach dem Lassen, um abzukühlen, oder Kühlen der gegossenen Mischung, bietet rektale Suppositorien oder vaginale Kügelchen mit einem Gewicht von 0,5 g bis 6 g.

13. Eine pharmazeutische Zusammensetzung nach einem der Ansprüche 1 bis 5 oder eine nach einem Verfahren nach einem der Ansprüche 6 bis 12 hergestellte pharmazeutische Zusammensetzung zur Verwendung als Arzneimittel zur Behandlung von Tumorerkrankungen.

## Revendications

1. Une composition pharmaceutique pour l'application rectale et vaginale, **caractérisée en ce qu'**elle contient, en tant que principe actif, un complexe de platine de formule II 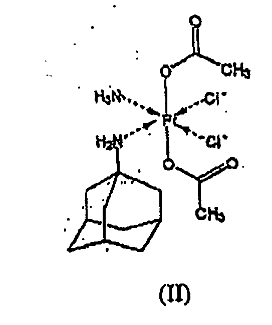 et une base gel-formante hydrophile comprenant de la gélatine, de l'eau et du glycérol.

2. La composition pharmaceutique selon la revendication 1, **caractérisée en ce qu'**elle comprend au moins un monosaccharide et/ou disaccharide et/ou polysaccharide.

3. La composition pharmaceutique selon la revendication 1 ou 2, **caractérisée en ce que** le principe actif a composition granulométrique telle que 100 % de particules du principe actif sont égales à ou plus petites que 100 micromètres, de preférence 80 % de particules du principe actif sont égales à ou plus petites que 50 micromètres.

4. La composition pharmaceutique selon l'une quelconque des revendications 1 à 3, **caractérisée en ce qu'**elle est dans la forme d'une unité de dosage comprenant 5 à 500 mg de principe actif, de manière avantageuse 20 à 200 mg de principe actif.

5. La composition pharmaceutique selon l'une quelconque des revendication 1 à 4, **caractérisée en ce qu'**elle est dans la forme d'un suppositoire ou d'une gélule vaginale.

6. Un procédé de la production d'une composition pharmaceutique selon l'une quelconque des revendications 1 à 5, **caractérisé en ce que** le principe actif est mélangé avec une base gel-formante hydrophile comprenant de la gélatine, de l'eau et du glycérol liquéfiée de manière thermique et le mélange obtenu est ensuite formé.

7. Le procédé selon la revendication 6, **caractérisé en ce qu'**un mélange du principe actif avec au moins un monosaccharide et/ou disaccharide et/ou polysaccharide, avec le(s)quel(s) le principe actif était préalablement broyé pour obtenir une taille désirée de particules, est mélangé avec une base gel-formante hydrophile liquéfiée de manière thermique.

8. Le procédé selon la revenidication 7, **caractérisé en ce que** la teneur en l'un au moins de monosaccharide et/ou disaccharide et/ou polysaccharide du mélange avec le principe actif destiné au broyage préalable est égale à ou plus grande que 20% de poids, de preférence égale jusqu'à 50% de poids, rapportée au poids du principe actif.

9. Un procédé selon la revendication 7 ou 8, **caractérisé en ce que** le principe actif est broyé avec au moins un monosaccharide et/ou disaccharide et/ou polysaccharide pour obtenir une composition granulométrique telle que 100 % de particules du principe actif sont égales à ou plus petites que 100 micromètres, de preférence 80 % de particules du principe actif sont égales à ou plus petites que 50 micromètres.

10. Un procédé selon l'une quelconque des revendications 6 à 9, **caracterisé en ce que** le mélange du principe actif avec une base gel-formante hydrophile liquéfiée de manière thermique est réalisé dans un mélangeur la surface intérieure duquel venant en cotact avec le mélange traité est faite en matériel inerte, de preférence en verre ou céramique, ou en matériel couvert de polytétrafluoréthylène ou en matériel autrement modifié de manière non-métalique.

11. Un procédé selon l'une quelconque des revendications 6 à 10, **caractérisé en ce que** le mélange obtenu par le brassage du principe actif avec une base gel-formante hydrophile liquéfiée de manière thermique est formé par le coulage en moule pour suppositoires rectals ou gélules vaginales et le mélange coulé en moule obtenu est ensuite refroidi de maninère spontanée ou forcée.

12. Le procédé selon la revendication 11, **caractérisé en ce que** le mélange obtenu par le brassage du principe actif avec une base gel-formante hydrophile liquéfiée de manière thermique est formé par le coulage en moule pour suppositoires rectals ou gélules vaginales et donne, after le refroidissement spontané ou forcé du mélange coulé en moule, les suppositoires rectals ou gélules vaginales ayant le poids compris entre 0,5 g et 6 g.

13. Une composition pharmaceutique selon l'une quelconque des revendications 1 à 5 ou une composition pharmaceutique préparée par un procédé selon l'une quelconque des revendications 6 à 12 pour l'utilisation à titre de médicament pour traiter les maladies tumorales.
